(19) Europäisches Patentamt
European Patent Office
Office européen des brevets

(11) **EP 4 400 054 A1**

(12) **EUROPEAN PATENT APPLICATION**

(43) Date of publication:
**17.07.2024   Bulletin 2024/29**

(21) Application number: **23151500.8**

(22) Date of filing: **13.01.2023**

(51) International Patent Classification (IPC):
**A61B 6/00** $^{(2024.01)}$

(52) Cooperative Patent Classification (CPC):
**A61B 6/54; A61B 6/469; A61B 6/481; A61B 6/488;
A61B 6/504**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **DESHPANDE, Hrishikesh Narayanrao
Eindhoven (NL)**

• **SCHMITT, Holger
Eindhoven (NL)**
• **GOTMAN, Shlomo
5656AG Eindhoven (NL)**
• **BERGTHOLDT, Martin
Eindhoven (NL)**
• **NETSCH, Thomas
Eindhoven (NL)**

(74) Representative: **Philips Intellectual Property &
Standards
High Tech Campus 52
5656 AG Eindhoven (NL)**

(54) **AUTOMATIC BOLUS ROI PLACEMENT USING 3D CT SURVIEWS**

(57)     System (FS) and related method for facilitating contrast-agent based tomographic imaging, The system may comprise an input interface (IN) for receiving a specification for an imaging operation of an imaging apparatus (IA) of the tomographic type, and a 3D surview image volume (V0) obtained by the imaging apparatus (IA) of a patient in a preparatory phase prior to a contrast agent assisted imaging phase. A segmentor (SEG) segments for a monitoring region, m-ROI, in the 3D surview image volume. The m-ROI is associated with a target anatomical feature as may be specified in the specification. An output interface (OUT) provides a reference location (z0) of the segmented m-ROI in the 3D surview volume for facilitating monitoring for presence of contrast agent in relation to the target anatomical feature.

FIG. 4

**Description**

FIELD OF THE INVENTION

The invention relates to a system for facilitating contrast-agent based tomographic imaging, to a training system for training, based on training data, a machine learning model for use in such a system, to a training data generator system, to related methods, to an imaging arrangement, to a computer program element, and to a computer readable medium.

BACKGROUND OF THE INVENTION

2D (two dimensional) projection surview image(s) acquired before diagnostic CT (computed tomography) imaging plays an important role in assisting clinical technologists ("user"), for example in proper patient positioning or in achieving optimal image quality.

In addition to conventional 2D surviews (such as frontal and sagittal), today it is possible to acquire 3D (reconstructed) surview images (an image volume) at clinically acceptable dose. They offer several advantages over conventional 2D surviews, and open up new possibilities for CT workflow optimization, which was not possible earlier.

It is common practice in CT imaging to perform a 2D surview scan covering the entire area of interest including an expected "bolus" tracking location. Bolus is a volume of administered contrast agent material such as Iodine. Contrast agent may be used in order to boost image contrast of weakly radiation absorbing anatomical structures of interest, such as cardiac vessels in cardiac imaging. The user then manually selects the z-position on such 2D surview(s), and the corresponding axial image, also known as a "locator", is acquired and reconstructed for definition ("placement") of a region of interest ("ROI") for bolus tracking or monitoring for bolus arrival. Bolus travels with blood flow and accumulates, for a time, at the anatomy of interest. It is an object to trigger imaging at the right moment to capture imagery at high contrast before bolus wash-out.

Multiple locator scans may be needed to find proper location for the tracking ROI. The ROI to be captured for tracking the bolus is placed within this locator slice, and is monitored for the arrival of the contrast agent bolus, by repeated acquisition of this single axial slice. The subsequent diagnostic CTA (CT angiography) acquisition is triggered when a threshold value of contrast induced density is reached within this ROI.

A major disadvantage of bolus tracking using conventional 2D surview images, or of bolus tracking in general, is that planning of bolus tracking during the scan procedure is complex and relies heavily on clinical user's skill and experience. The process is error prone. For example, there are several manual interventions needed by user, such as manually selecting the z-slice, placing an ROI on the acquired locator slice etc. If there are human errors in the localization of anatomical structures and placing the ROI in the localizer image, the diagnostic CT scan may be started at a suboptimal time. This could subsequently result in poor image quality and wrong diagnosis, and could lead to unnecessary re-administration of contrast agent and image retakes, with potential side-effects on patient and/or medical staff. Also, the manual localization process is subject to intra- and inter-individual variability. Another disadvantage is the need to acquire special locator scans(s) which requires extra time and dose.

SUMMARY OF THE INVENTION

There may be a need for more efficient operation of an imaging apparatus. In particular, there may be a need for establishing reliably the correct timing for triggering an imaging operation for such an imaging apparatus.

An object of the present invention is achieved by the subject matter of the independent claims where further embodiments are incorporated in the dependent claims. It should be noted that the following described aspect of the invention equally applies to the training system for training, to the training data generator system, to the related methods, to the imaging arrangement, to the computer program element, and to the computer readable medium.

According to a first aspect of the invention there is provided a system for facilitating contrast-agent based tomographic imaging, the system comprising, when in use:

an input interface for receiving a specification for an imaging operation of an imaging apparatus of the tomographic type, and a 3D surview image volume obtained by the imaging apparatus of at least a part of a patient in a preparatory phase prior to a contrast agent assisted imaging phase;
a segmentor configured to segment for a monitoring region ("m-ROI"), in the 3D surview image volume, the said m-ROI associated to a target anatomical feature, for example as specified in the specification; and
an output interface to provide a reference location of the segmented m-ROI in the 3D surview volume for facilitating monitoring for presence of contrast agent in relation to the target anatomical feature.

The specification may be part of an imaging protocol for example. The specification may specify the target anatomical feature which is the purpose for the imaging. Based on medical knowledge, the specification may define the kind of anatomical landmark(s) or other auxiliary anatomical features (preferably a part of a (blood) vessel) that may serve as the m-ROI as a function of the intended target anatomical feature. The segmentor turns this medical and anatomical knowledge into a concrete location definition for the (one or more) m-ROI in the given 3D surview image/volume, to so assist medical user in this task.

The target anatomical feature may relate to an organ/anatomy, part of organ/anatomy, group of organs/ anatomies, tissue type(s), etc. The target anatomical feature may be a vessel, or a part/section thereof. The m-ROI is in general different from target anatomical feature, located elsewhere, preferably upstream of target anatomical feature in respect of blood flow. The m-ROI may be a specific part of a vessel, a section thereof, etc. The vessel may supply the target anatomical feature.

In embodiments, the system comprises a controller configured to instruct imaging apparatus to acquire a first set of projection data whilst contrast agent propagates in patient, and comprising a reconstructor to reconstruct, based on the projection data and at a first image quality, sectional tracker imagery in a plane passing through the reference location.

In embodiments, an in-image monitoring unit is configured to perform such monitoring based on the tracker imagery in an image-neighborhood in the tracker imagery, the said image-neighborhood based on the reference location. The image-neighborhood is preferably produced by the facilitator or may be sourced otherwise. The size of the image-neighborhood may be pre-defined or adaptable

Preferably it is the facilitator that produces the image-neighborhood. For example, the said image-neighborhood is defined in a synthesized tracker image (the "locator") synthesized from the surview image, and such image-neighborhood is then used by the in-image monitoring unit for monitoring in the said reconstructed tracker imagery. Thus, in the proposed system, the locator may be obtained synthesized off the surview image. Thus, no dose needs to be spent on locator. This affords dosage savings for patient and/or staff. The in-image monitoring unit may be part of the system.

In embodiments, a controller of the system is configured to instruct imaging apparatus to acquire, at a second image quality higher than the first image quality, a second set of projection data upon the in-image monitoring unit issuing a trigger signal based on the monitored one or more image values in the image-neighborhood.

In embodiments, the said reference location is based on at least one reference point in relation to the segmentation of the m-ROI.

In embodiments, the said at least one reference point includes a centroid of the segmentation of the m-ROI.

In embodiments, the system includes an adjuster for adjusting any one of location, orientation and/or spatial extent of the said in-image neighborhood. The adjusting may be upon user request and user may control adjustment by a user interface ("UI").

In embodiments, said adjuster is operable in response to an instruction receivable via a user interface configured to allow a user to request adjusting any one or more of: i) the said in-image neighborhood, ii) the reference location, iii) the reference point, iv) the segmentation of the m-ROI.

In embodiments, the adjusting by adjuster is based on a latency in relation to the imaging phase. The latency may relate to machine-sided latency, that is, how quickly the imaging apparatus can start acquiring, delays in reconstruction from projection data, etc. Latency may also relate to patent-sided aspects, such as bolus hemodynamics, how quickly the bolus propagates through patent's vessel system.

In embodiments, the segmented m-ROI (s) is spatially co-extensive with a geometrical aspect of the target anatomical feature.

In embodiments, the segmentor is based on a trained machine-learning model. The facilitator may be used after training, in deployment to testing, as required.

In embodiments, the model is of the artificial neural network type.

In embodiments, the model is configured for multi-scale processing of image information. This allows better processing of even poor image quality (high noise) imagery, such as is the case with low dose surview imagery.

The proposed system takes as input for processing the 3D surview image volume (a tomographic reconstruction), as opposed to a mere 2D projection surview image. This 3D information is harnessed herein to allow the system to, preferably automatically, if not fully automatically, select the reference location (eg, a z-position), and define the neighbored for the m-ROI bolus on an axial (or in any other view) slice synthesized at the corresponding z-location from the 3D surview image. The neighborhood defines location and, preferably spatial extent, of the sought after m-ROI. Thus, z-slice selection and m-ROI neighborhood placement can be done in one/single step, using the segmentation of the 3D bolus m-ROI. Thus, m-ROI definition and its representation in the tracker imagery is achieved via image segmentation only. This greatly simplifies workflow for user. More patients can be imaged safely and quickly.

Preferably, user has option to still adjust/modify the automatically proposed reference location (z-position) and hence the slice selection. User can any one of move, or adapt the size of the automatically placed bolus m-ROI neighborhood.

The user requested adjustment may be embedded in a dynamic feedback mechanism. Thus, a user requested adjustment of any of the components of the m-ROI, such as z-location, will automatically update the corresponding axial slice selection and adapt the bolus ROI neighborhood on the new/updated slice, with the neighborhood placed on this new slice. The ability of the system to place m-ROIs for various locations via 3D bolus segmentation and the afforded option for user to adapt the related slice selection and neighborhood placement at both stages, allows for a highly efficient clinical workflow.

Furthermore, no image registration is required in the proposed system and method (see below), as the image segmentation is done in embodiments via the machine learning model trained end-to-end. Thus, the machine learning model maps input (the surview image) into the segmentation in the same surview image. A single model can be used that outputs the z-location and this automatically causes definition of the neighborhood of the bolus m-ROI in the synthesized tracker image for a guided image acquisition. Any geometrical shape that is deemed apt may be used to define m-ROI#s location/extent, such as a circle, ellipse, square, or any other shape that may be chosen for the neighborhood.

The user can intervene at any of these two steps and adapt the workflow as they wish. The proposed system is configured to follow the CT imaging workflow more closely, but in a more efficient manner. Thus, to the user is still displayed the automatic results for both, z-slice selection on the coronal view and bolus tracking ROI on the axial slice. Both outputs, the z-position and the neighborhood for the m-ROI, are found by the same segmentation of 3D surview volume and post-processing the segmentation map to obtain the refence point, and, based on this, the selected z-slice form the surview image.

The system may be used to produce z-slice selection on a coronal view of the surview image, whilst the bolus tracking m-ROI is represented on an axial tracker imagery (slice), to resemble closely current CT workflow practice. However, any combination of such views are possible.

The model is preferably trained on labeled ground truth data, as this facilitates the single step operation of (i) z-location in the surview image and (ii) placement of neighborhood for bolus m-ROI on the synthesized locator slice. The definition of the neighborhood may be done in image space, or in a transformed space, such as in a distance transform of the synthesized tracker image, or in any of the follow up tracker images reconstructed from newly acquired projection imagery.

Other advantages and benefits of the proposed system and method (see below) includes greater consistency of performance across clinical user cases. For example, spatial distances from the bolus m-ROI to the target organ of the CT angiography or other imaging session might well differ from patient-to-patient. By using 3D surviews as proposed herein, it is possible to place the m-ROIs consistently at a certain pre-defined distance upstream to the target structure to be imaged. Having latency information available, which can be easily ascertained from tests or vendor specifications, the scan length and timing could be optimized to be as short as possible by placing the m-ROI close to target anatomy and consistently across a patient cohort. Such latency information may include time periods on how long it takes to start the scanner (to effect the acquisition operation) and how long it takes for the contrast agent to reach the target anatomy. The later can be estimated from medical knowledge or patient characteristics (eg, height, BMI), average blood flow data, etc.

The system and related methods (see below) are mainly envisaged herein for use in the medical realm. However, the principles described herein may also be used in fields of endeavor outside the medical field, such as in contrast agent (eg, dye) assisted examination of inaccessible hydraulic or plumbing systems, or in hydrological exams to understand subterranean water movements, etc. As is the case for medical imaging, t the system and method allow reducing wear and tear effects on imaging equipment as they can be used with better timing to trigger acquisitions at the instant of best image harvest, thus avoiding repeated imaging.

In another aspect there is provided a training system for training, based on training data, the said machine learning model.

In another aspect there is provided a training data generator system configured to generate training data based on which training system is to train the machine learning model.

In embodiments, the training data generator system is configured to facilitate generating the training date by inclusion of annotations in prior imagery, wherein the annotations are indicative of location and respective spatial extent of sample m-ROIs in the prior imagery.

In another aspect there is provided a computer-implemented method for facilitating contrast-agent based tomographic imaging, comprising:

receiving a specification for an imaging operation of an imaging apparatus of the tomographic type, and a 3D surview image volume obtained by the imaging apparatus of at least a part of a patient in a preparatory phase prior to a contrast agent assisted imaging phase;
segmenting for a monitoring region, m-ROI, in the 3D surview image volume, the said m-ROI associated to a target anatomical feature. The target anatomical feature may be specified in the specification for example.

The method may further comprise: providing a reference location of the segmented m-ROI in the 3D surview volume for facilitating monitoring for presence of contrast agent in relation to the target anatomical feature.

In another aspect there is provided a computer-implemented method of training, based on training data, the said machine learning model.

In another aspect there is provided a computer-implemented method of generating training data based on which training system may train the machine learning model.

In embodiments, the computer-implemented method includes receiving definition of seed markers for an existing surview image, and generating, based on the seed marker, a convex hull including all such seed markers, wherein the voxels enclosed by the convex hull represent an annotation for an instance of a monitoring region.

The seed markers may be configured geometrically as circles, ellipses, preferably any shape that can be made to fit at least approximately to follow at least partly and locally contours of the segmentation.

In another aspect there is provided there is provided an imaging arrangement comprising the system of any one of the preceding claims, and one of more of: the imaging apparatus and a contrast agent administration apparatus for administering contrast agent.

Any of the above-mentioned system may be provided on a suitably configured one or more computing systems.

In another aspect there is provided a computer program element, which, when being executed by at least one processing unit, is adapted to cause the processing unit to perform any given one of the above mentioned methods.

In yet another aspect there is provided at least one computer readable medium having stored thereon the program element, or having stored thereon the machine learning model.

"*user*" relates to a person, such as medical personnel or other, operating the imaging apparatus or overseeing the imaging procedure. In other words, the user is in general not the patient.

"*surview image*" is used herein is a 3D image volume obtained at lower radiation dose cost and at a lager field-of-view (FOV) than dose cost and FOV for and of a subsequent diagnostic image volume. The diagnostic image volume is required to accomplish or support a medical task, such as diagnosis, therapy, planning or other. The surview image is not used for such task. The FOV of the surview image volume includes not only the anatomy of interest, which is the very object of the medical task, but in general also captures other landmark anatomies that have only an auxiliary function for the imaging, but in general not for the medical task. The FOV may be a whole-body scan, but this needn't be so in all cases. In general, the FOV of the surview image captures larger anatomic section, such as abdominal, thorax, etc.

"*z-position*" or similar terminology as used herein relates to one example of a reference location in image domain as envisaged herein for definition of the tracker imagery. "z-position" in general relates to position on one (the "Z-axis") of the 3 spatial coordinate axes that span image domain. In general, this Z-axis corresponds to the rotation axis of rotational tomographic imagers, or to the imaginary rotation axis in imagers of $5^{th}$ generation scanners. However, the said Z-axis may differ from the rotation axis for reformatted surview volumes, also envisaged herein.

In general, the term "*machine learning*" includes a computerized arrangement (or module) that implements a machine learning ("ML") algorithm. Some such ML algorithms operate to adjust a machine learning model that is configured to perform ("learn") a task. Other ML operate direct on training data, not necessarily using such as model. This adjusting or updating of training data corpus is called "training". In general task performance by the ML module may improve measurably, with training experience. Training experience may include suitable training data and exposure of the model to such data. Task performance may improve the better the data represents the task to be learned. "*Training experience helps improve performance if the training data well represents a distribution of examples over which the final system performance is measured*". The performance may be measured by objective tests based on output produced by the module in response to feeding the module with test data. The performance may be defined in terms of a certain error rate to be achieved for the given test data. See for example, T. M. Mitchell, "Machine Learning", page 2, section 1.1, page 6 section 1.2.1, McGraw-Hill, 1997. Some machine learning approaches are based on back-propagation algorithms in various forms, some based on gradient methods.

"*image processing*" relates to type or manner or processing in relation to information as captured by imagery. However, in this connection, it is observed that any processing of imagery envisaged and described herein, in whichever embodiment and configuration or setup, includes, not only processing in image domain of image values, etc, but such reference specifically include processing in transform domain, such as frequency domain, or any other domain in which the imagery V may be transformed first, and at least a part of the processing takes place there, with optional re-transforming into image/class map domain, as required. Transforms envisaged herein include any Fourier based transform (Laplace transform, Discrete Cosine transform, etc), Wavelet-transform (such as Haar transforms), Hilbert transforms , but also Distance Transforms, and others still.

BRIEF DESCRIPTION OF THE DRAWINGS

Exemplary embodiments of the invention will now be described with reference to the following drawings, which, unless stated otherwise, are not to scale, wherein:

Fig. 1 shows a schematic block diagram of a medical imaging arrangement;
Fig. 2 illustrates a contrast agent-based imaging protocol;
Fig. 3 shows steps of a facilitator system for facilitating contrast agent assisted tomographic imaging;
Fig. 4 shows a block diagram with more details of the facilitator system;
Fig. 5 shows imagery generatable by the facilitator system;
Fig. 6 shows a machine learning model that may be used in embodiments of a segmentor of the facilitator system;
Fig. 7 shows a training system for training a machine learning model;
Fig. 8 shows a flow chart of a computer-implemented method for facilitating contrast agent assisted tomographic imaging; and
Fig. 9 shows a flow chart of a computer-implemented method of training a machine learning model.

DETAILED DESCRIPTION OF EMBODIMENTS

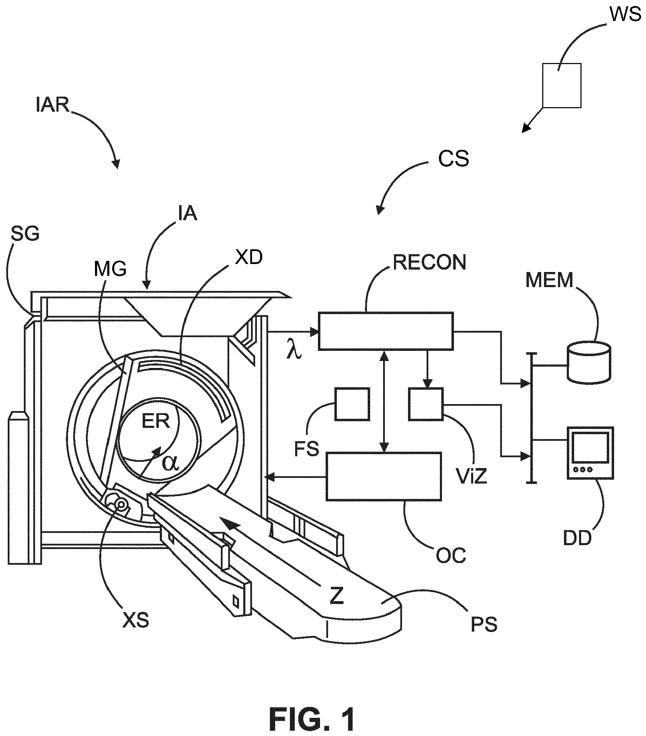

Reference is first made to Fig. 1 which shows a schematic block diagram of a medical imaging arrangement MAR as envisaged herein in embodiments. The arrangement IAR may include a medical imaging apparatus IA ("imager" for short), preferably of the tomographic X-ray based type, Thus the imager may be computed tomography (CT) scanner, but interventional systems such as those of the C-arm/U-arm-type are not excluded herein. Other tomographic modalities such as MRI, PET and others still are not excluded herein in some other embodiments. The medical imaging arrangement is preferably envisaged for contrast agent-based imaging protocols, such as angiography.

The arrangement IAR further includes a computing system CS which is broadly operable to process data, including image data, as provide by the imager. The computing system may further allow controlling operation of the imager. The computing system may be remotely located from the imaging apparatus IA, or may located close thereto, such as integrated into an operator console CS from which a user can operate the imaging apparatus, in particular can control an imaging operation to obtain medical imagery for diagnosis, treatment, planning (such as in radiation therapy, or other), etc.

Broadly, and as will be explained more fully below, the computing system CS includes a facilitator system FS that may be used to establish the right timing to trigger imaging operation to ensure good quality (contrasted) imagery is obtained.

The computing system CS., in particular its facilitator system FS, may be implemented as a Cloud solution, run on one or more servers. The imaging apparatus IA may be installed in a clinical facility, such as in a hospital for example. The computing system may be installed or used in a control room next to the imaging room where the imager IA is located. In some embodiments the computing system may be integrated into the imager IA. The imager IA may be communicatively coupled via a wired or wireless (or partly both) communication channel CC to computing system CS. The computing system CS may be arranged as a stationary computing system, such as a desktop computer, or as said server, or may be arranged as a mobile device, such as a laptop, smartphone, tablet, etc.

Before explain operation of the facilitator system FS in more details, reference is first mode to components of the imager IA which will the following explanations in relation to the facilitator system FS.

The imaging apparatus IA is operable to generate, in particular acquire, projection data $\lambda$ which are forwarded through communication channel to the computing system CS for reconstruction into tomographic (sectional) images. The computing system CS runs one or more reconstructor units RECON, which implement one or plural reconstruction algorithms. In general, reconstruction algorithms implement a mapping that maps projection data $\lambda$ located in projection domain into image domain. Image domain is a portion of 3D space and is located in the examination region ER of the imaging apparatus, whilst the projection domain is in 2D, and is located at an (X-ray) detector XD of the imaging apparatus IA.

As said, the imaging apparatus IA is preferably of the tomographic type, and is preferably configured for multi-directional projection image acquisition. The imaging apparatus IA is thus capable of acquiring projection images $\lambda$ along different projection directions $\alpha$ relative to the examination region ER, and thus the anatomical region ("ROI") of interest of the patient. Acquisition be done in embodiments by a rotational system where at least the X-ray source XS is arranged in a moveable gantry MG.

The movable gantry (and with it the X-ray source XS in embodiments) is rotatable in a stationary gantry SG around the examination region ER in which the patient/ROI resides during imaging. Opposite the X-ray source in the movable gantry there is the X-ray detector XD which may rotate with the gantry and X-ray source around the examination region ER to realize the different projection directions $\alpha$.

As schematically indicated in Fig. 1, patient's longitudinal or imaging axis Z may extend into the examination region ER during imaging. Patient PAT may lie on a patient support PS, such as a bed, which is positioned at least partly in the examination region ER during imaging. In some, but not all embodiments, helical imaging protocols are envisaged herein where there is a relative lateral motion along the longitudinal axis Z between X-ray source XS and the patient PAT. For example, the patient support PS may be advanced through the examination region ER during the multi-directional projection image acquisition, for example during rotation of the X-ray source XS around the patient.

The CT scanner setup as illustrated in the Fig. 1 is merely according to one embodiment, and other tomographic imaging equipment, such as C-arm or U-arm scanners, Cone beam CT arrangements, mammographic imagers, etc, are not excluded herein. C-arm cone beam imagers are preferred herein in some embodiments. In addition, the multi-directional acquisition capability may not necessarily result from a rotational system such as shown in Fig. 1. Non-rotational imaging systems are also envisaged, such as in CT scanners of 4th or 5th generation, where multiple X-ray sources are arranged around the examination region in a source annulus for example. In addition or instead, the detector XD may be arranged as a detector annulus around the examination region. Thus, in such systems there is no rotation of the X-ray source XS or detector XD, or of both.

There may be an operator console OC through which a user, such as medical personnel, controls imaging operation. For example, user may request initiating the image acquisition or may request reconstruction or other operations, or may initiate transmission of data to the computing system CS, or may halt such transmission as required.

During imaging, an X-ray beam XB issues forth, along the different projection directions $\alpha$, from a focal spot of the X-ray source(s) XS. The beam XB passes through the examination region with the patient in it. The x-radiation interacts with patient tissue. The X-ray beam XB is modified as a result of this interaction. In general, such X-ray beam XB modification includes attenuation and scattering of the original incoming X-ray beam. The modified X-radiation is then detected as a spatial distribution of various intensities at X-ray sensitive pixels of the detector XD.

It is not necessary herein to acquire projection images $\lambda$ over a full $360^0$ angular range around the examination region ER. Acquisition over a partial angular range such as a range of 270°, 180°, or even less may be sufficient. The X-ray detector is preferably configured for acquisition of 2D projection images with rows and columns of intensity values as registered by the detector pixels. That is, the detector pixels themselves may be arranged in a matrix layout. Such a 2D layout may be used in divergent imaging geometries, such as cone or fan beam or others. However, one dimensional detector pixel layouts (such as along a single line) are not excluded herein, and neither are parallel beam geometries.

The reconstructor RECON implements one or more reconstruction algorithms to process the projection imagery. Specifically, the reconstructor RECON may compute sectional image V of the examination region (with the patient in it) for diagnostic, therapeutic or other purposes. The reconstructor RECON may be able to produce sectional volumetric image data ("image volume(s)") V. However, this does not exclude producing a single image slice in the examination region as required. Thus, the reconstructed imagery may be denoted herein as V which may include the whole volume, a partial volume or a specific section therethrough. Volumetric reconstruction may be facilitated by the helical movement and/or the 2D layout of the X-ray detector XD.

The reconstructed volumetric imagery $V$ may be stored in a memory MEM or may be otherwise processed, as required. The reconstructed volumetric imagery $V$ may be visualized by a visualizer VIZ. The visualizer VIZ may generate a graphics display of the volume or a given slice. The graphics display may be displayed on a display device DD. The visualizer VIZ may map a section of image volume V, or the image volume V as a whole, onto a grey value or a color palette. The visualizer VIZ may control, via suitable interfaces, video circuitry to effect display of the graphics display on the display device DD. In addition or instead of so displaying, the reconstructed imagery $V$ may be stored in a memory for later review or for other types of processing. Such a memory may include an image repository, such as a database (eg, PACS), or other (preferably) non-volatile data storage arrangement.

The reconstructed volume imagery $V$ may be manipulated, for example by reformatting, to define cross-sectional planes that are different from the sectional plane defined by the imaging geometry. Such reformatting may allow medical user to better discern tissue type or anatomic details of the internals of the patient, in dependence on the medical purpose at hand, such as for diagnosis or preparation for some type of treatment, etc.

The so reconstructed volume $V$ may be referred to herein as the target image acquired in a target or operational phase of the contrast agent assisted imaging procedure. This reconstructed image V is envisaged as a diagnostic image that represents the target anatomical feature TAF, such as a target anatomy, an organ, part of an organ, or group of organs, different tissue types, etc, and at sufficient contrast to safely inform therapeutic or diagnostic decisions, or other medical decisions such as to have further imaging sessions done, possibly using other imaging modalities, or other tasks (tests, etc) that need be done in light of the imagery.

The (target) projection imagery $\lambda$, from which the target volume V is reconstructed, is acquired at a sufficient dosage by suitably controlling via operator console OC the dose to be used. This may be done by controlling the voltage and/or amperage setting of the tube of the x-ray source XS to ensure a certain diagnostic image quality. As mentioned earlier, the imaging protocol envisaged herein is preferably contrast agent based to ensure that the target anatomical feature TAF, that may be natively of low radio- opacity, can still be imaged at sufficient contrast. Such target projection imagery acquired at sufficiently high dose, may also be referred to herein as the diagnostic projection imagery $\lambda$ in order to follow established terminology. However, this naming convention is not to preclude that this projection data $\lambda$ and its reconstruction V is to be used for tasks other than diagnostics, such as for therapy (in Cath Labs, etc), planning or any other task.

The target anatomical feature TAF relates to the medical purpose of the imaging. Thus, if patient's liver need examining, the target anatomical feature TAF is the liver: it is the liver that is the purpose and object of an abdominal scan to be done.

Each target anatomical feature TAF is in generally associated with an imaging protocol, a set of specifications, that stipulate, preferably as a function of patient bio-characteristics (age, weight, sex, height, BMI, medical record, etc), certain preferred imaging settings, the required image contrast to be achieved, the radiation dose to use for the target anatomical feature TAF for the given purpose, voltage/amperage settings of source X to use, collimation, etc. In short, imaging protocols encapsulate the medical knowledge for any given imaging task, purpose, target anatomical feature TAF, etc.

Before acquisition of such diagnostic projection imagery $\lambda$ , and for anatomical feature TAF of such low radio-opacity, a volume of contrast agent CA, such as Iodine or other suitable material, is administered to the patient PAT via an administration device ADA (such as a pump). This volume (sometimes called a "bolus") of contrast agent CA then propagates through the patient with blood flow, and accumulates at the target anatomical feature TAF. Ideally, once sufficient contrast agent CA has so accumulated at the targeted anatomical feature TAF, only then should the diagnostic projection imagery at the diagnostic quality be acquired. Thus, the timing for the acquisition by imager IA of the diagnostic projection imagery $\lambda$ for the target image V is an important consideration as it must be ensured that the concentration of the contrast agent at the target anatomical feature of interest is sufficient. Only then can the reconstructable image domain target volume V be expected to have the required IQ (image quality) as per the protocol or as otherwise prescribed image contrast. If this is not the case, retakes may be required which is to be avoided due to cost, time efforts, increased dose exposure, machine wear (in particular of the anode disk of tube XS), etc. Hence it is a consideration herein to "get" the diagnostic acquisition timing "right first time".

The facilitator system FS envisaged herein facilitates such acquisition at the right timing in contrast assisted imaging protocols. The facilitator FS ensures that, reproducibly and reliably, the diagnostic projection image acquisition is triggered at the right moment when sufficient contrast agent has in fact accumulated at the target anatomical feature TAF.

The facilitator system operates across two phases, an exploratory or preparatory phase PP, and a monitoring phase, which both preceding the target phase at which the target volume V is obtained. All such phases are preceded by an initial phase in which an initial surview image V0 is obtained. This initial image V0 is preferably a 3D (image domain) surview volume, itself reconstructed from a first set of projection imagery. But this first /initial set of projection imagery $\lambda 0$ is acquired at a lower image quality than the image quality of the projection imagery $\lambda$ to be acquired later in the target phase and to be used for reconstruction of target image V. In particular, when acquiring $\lambda 0$, lower radiation dose is incurred as compared to the later dose that will fall due for the diagnostic scan to acquire the diagnostic projection imagery $\lambda$. This is to save dose on the patient PAT, and also because the 3D surview volume V0 serves an entirely different purpose than does the target volume V: the purpose of the surview volume/image V0 is essentially one of navigation, as will be expanded upon herein: the surview image V0 is used to find a suitable location to monitor for bolus arrival to ensure imaging for the target anatomical feature TAF commences at the right moment in time, so that target volume V has the expected diagnostic-grade contrast.

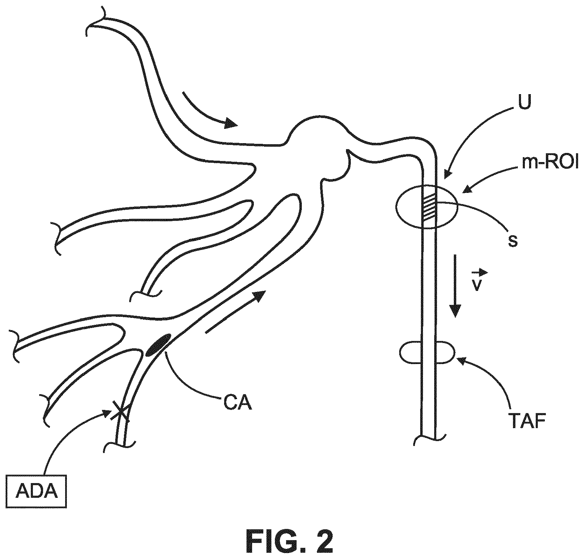

The contrast agent assisted imaging protocol is illustrated schematically in Fig. 2, to which reference is now made, before explaining operation the facilitator system FS in more detail.

As observed earlier, contrast agent CA is to boost image contrast for target structure TAF with poor native radio-opacity. In this connection, and referring now more specifically to Fig. 2, this is a schematic illustration of a part of a blood vessel into which, at an access point (shown at "*X*"), bolus CA is administered by the said contrast agent administration apparatus ADA or other.

A blood vessel, such as a cardiac artery or vein of interest in cardiac imaging, is soft tissue and hence has, as such, poor radio-opacity. It is thus represented at low contrast if one were to use non-contrasted scans. The volume of contrast agent CA travels with the blood flow and propagates therethrough until a concentration of contrast agent accumulates at the target feature TAF at which point the target phase can commence, where the projection imagery for the target volume V at a higher image quality is acquired.

Preferably upstream of the target anatomical feature TAF (blood flow direction is indicated in Fig. 2 by vector $\vec{v}$), a monitoring region m-ROI is defined by an in-image neighborhood U as will be explained in more detail later on. This neighborhood U is based on a 3D segmentation s as found in the initial surview image V0. The neighborhood U and the segmentation s may relate to an anatomical landmark at which the monitoring of the contrast agent concentration is to take place. That is, the monitoring region m-ROI may be defined in terms of an anatomic landmark as per medical knowledge initially, although the exact, specific location in the given volume is provided by the facilitator FS.

Thus, the concentration of contrast agent CA is preferably monitored upstream the actual target anatomical feature TAF in order to account for image acquisition latencies for example. Once the concentration as monitored for at the monitoring region m-ROI has reached a certain minimum concentration, as may be measurable by thresholding in a time ($t$) series of tracker imagery $r(t)$ (to be explored more fully below), acquisition of the now diagnostic projection data $\lambda$ for the target volume V may commence as by that time, thanks to the blood flow, the concentration at target anatomical feature TAF can be expected to have reached an acceptable minimum concentration.

The spatial distance between the monitoring region m-ROI and the actual targeted anatomical feature TAF is mainly based on clinical knowledge and/or is determined by characteristics of the patient, knowledge of blood flow velocity, etc. Such medical context information including the m-ROI-TAF distance, which landmarks to use for monitoring region m-ROI, etc, may all be encoded in the mentioned imaging protocol/specification for the imaging task/purpose at hand.

Broadly, facilitator system FS is configured to quickly, and as said reliably and reproducibly, but also accurately, find the correct monitoring region m-ROI for a given patient and target anatomical feature TAF. Preferably, operation of the facilitator system FS is such that it integrates well into existing CT workflows. Whilst the facilitator system FS is envisaged to operate fully automatically and provide the monitoring region m-ROI without further/any user input (other than specifying the imaging protocol or target anatomical feature TAF), such additional user input is specifically envisaged in some embodiments. In such user-interaction inviting embodiments, it is specifically provisioned for that certain components of the monitoring region finding procedure can specifically be adjusted by the clinical user at their pleasure, through a suitable user interface UI, such as a graphical user interface (GUI). User request for such adjustment may be passed on to the system SYS via touch screen, pointer tool (computer mouse, stylus), etc. Such user-interaction is preferably envisaged herein as a dynamic real-time experience: upon such user-called-for adjustments in relation to the found monitoring region m-ROI, a re-computation of related components is triggered, and their display may be updated, as many times as the user calls for such changes or adjustments.

As will be explained in more detail below, a machine learning model M may be used in implementing the facilitator system FS. The facilitator system FS allows finding the right moment $t=t_0$ for the target phase to commence and to control the imaging apparatus IA, to reliably acquire the higher quality projection imagery $\lambda$ from which the target volume V can be reconstructed.

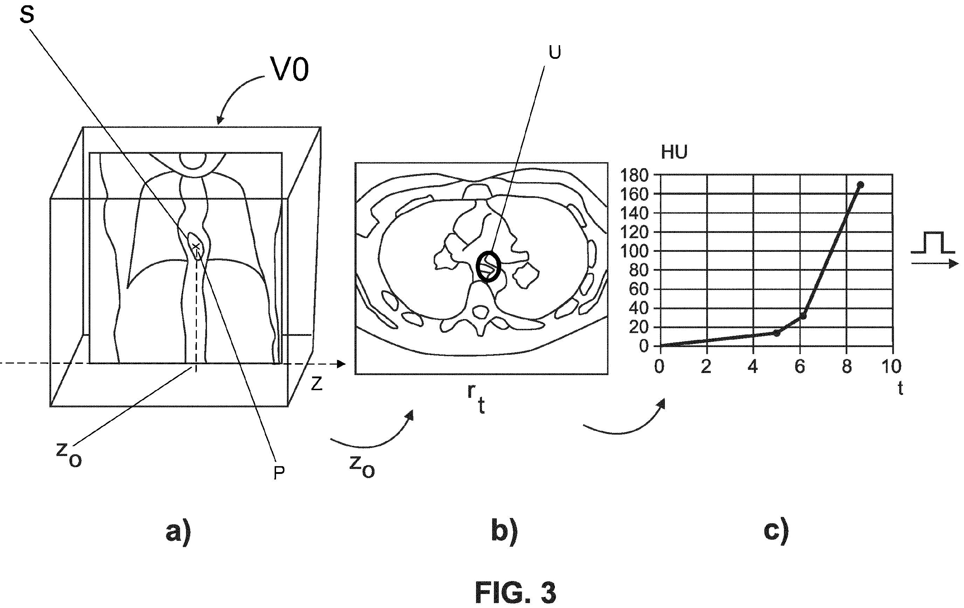

Schematic Fig. 3 illustrates some basic operational aspects of the proposed facilitator system FS. Specifically, and as illustrated at a), the initial 3D surview volume $V_0$ is segmented for suitable one or more anatomical landmarks that is to serve as the bolus monitoring region m-ROI.

Based on the segmentation s, a reference location z0 in the volume is identified which may, for example, be a set location along the rotation/imaging axis Z of the imager IA.

A time series of tracker imagery $r_t$ is then reconstructed from respective sets of low dose projection imagery acquired at suitable sampling interval around reference location z0.

In some or each such tracker images r(t), a monitoring neighborhood U (shown as a small circle) at b) is defined automatically, based on the reference location z0. In the mentioned user-interactive embodiments, an event handler may be used to allow user to change z0 or U, as required. Thus, it will be understood that the monitoring region m-ROI may be defined by two elements: its location in 3D as given by the reference location z0, and the spatial extent for monitoring purposes, as it is represented in the tracker imagery $rt$, which representation is defined by the neighborhood $U$.

In the series of tracker imagery $r(t)$, the concentration of the contrast agent arrival is monitored after administration of bolus. An example is shown as a contrast curve $c^U(t)$ at c) for a given tracker image and image neighborhood $U$. In such curve, contrast value $HU$ (in Hounsfield units) in neighborhood $U$ is recorded over time $t$. CA concentration, and hence contrast, is expected to increase over time in a ramp-up phase, to then plateau and reach saturation in plateau phase, and to then drop off in drop-op phase, as the contrast agent washes out. Only the ramp-up phase is shown in Fig. 3.

An image value-based thresholding policy may be used in the monitoring at neighborhood U to trigger acquisition signal for acquisition of the diagnostic projection imagery from which the target volume V is reconstructable. This acquisition of $\lambda$ should be triggered after the contrast agent concentration (or HU value in the contrast curve c) has reached a certain minimum value which may be lower than the expected maximum value at the target anatomical feature TFA, as this is expected to be located slightly downstream of the monitoring region m-ROI/U. Instead of basing the above-described thresholding on monitoring absolute HU values, gradients of HU values may be monitored for instead, or in addition, as required.

However, in some embodiments where latency is not an issue it may be that the monitoring region and the targeted anatomical feature are coinciding in which case the thresholding can be done so that the diagnostic projection image acquisition is triggered at the maximum of concentration values in plateau phase. The maximum may be found by using gradient based methods for example.

The sets of projection imagery acquired at the set sample rate from which the tracker imagery is reconstructed from, is equally of lower quality (lower dose) than the projection imagery $\lambda$ for the later diagnostic acquisition phase. For example, the image quality may be similar to that of the projection imagery acquired from which the surview image V0

was reconstructed.

The size (field-of-view ["FOV"]) of the surview (also called "scout") image volume V0 is preferably so chosen that it includes not only the target feature TAF, but also at least one of the anatomical landmarks at which the bolus monitoring is supposed to take place. If in doubt, the surview scan may be a whole-body scan, although this will not always be necessary as in some cases a scan over an applicable body region, such as an abdominal scan, head scan, thorax scan, leg scan, etc, may be sufficient.

Whilst contrast is usually very poor in such surview image V0 on account of the low dose, it may nevertheless be sufficient to locate broadly the said landmarks for m-ROI and, optionally, the targeted anatomical feature TAF. The later may also be marked up manually by user (see below).

Once the reference location Z0 is found by system FS based on the segmentation s, the imaging geometry may need to be adjusted for example by moving the patient table relative to the gantry so that the tracker imagery around the reference location can be obtained by reconstruction from the series of projection imagery acquired at the mentioned sampling intervals. However, in preferred embodiments, such a repositioning of table or gantry is not necessary, as the distances between target anatomical feature and monitoring location is roughly known, and the surview volume V0 is preferably obtained from the outset to cover a sufficiently large volume. For example, the FOV of the surview scout volume V0 may be linked with an auto-plan, so in general there is no need of a very large FOV surview to collect the anatomical context information, for example for the detection of landmarks, etc. Such auto-plan is a facility that allows detection of the target anatomical feature TAF in the surview image V0, or allows defining the FOV to be used. The auto-plan facility may be implemented by suitable image processing, for example again by segmentation, if required. However, detection and knowledge of location of the TAF may also be provided manually by user, or by whichever means, machine learning based or not. In general, it is assumed herein that the location of the target anatomical feature TAF is known, the main focus in this disclosure being the reliably locating of the monitoring region m-ROI.

As will be explored in more detail below, the segmentation s is a 3D segmentation, that is, it is defined by a 3D sub-volume in the surview image, $s \subset V_0$. This sub-volume having spatial extensions in all three spatial directions (X,Y,Z) is preferably anatomy aware in that it corresponds, in particular conforms, in shape, size and orientation to the spatial structure of the anatomy at that location. For example, the segmentation s may conform to the spatial structure of the anatomic landmark that is associated with the target anatomy. The segmentation may thus follow at least in part the anatomical/tissue boundary of the landmark. This allows user to quickly verify visually at a glance whether the system FS-proposed segmentation makes medical sense.

The, in general, relatively small, segmented anatomy s may be used for the definition of the reference location z0, and, separate therefrom, the definition of the neighborhood U in the tracker slice imagery that pass through the said reference location z0. As mentioned, the reference location z0 may be situated on the imaging axis Z. However, this is not a necessity herein as the initial surview volume V0 may also be provided as a reformat, and the reference location z0 may be a point on any geometrical line for the said reformat, and hence different from the rotational/imaging axis Z.

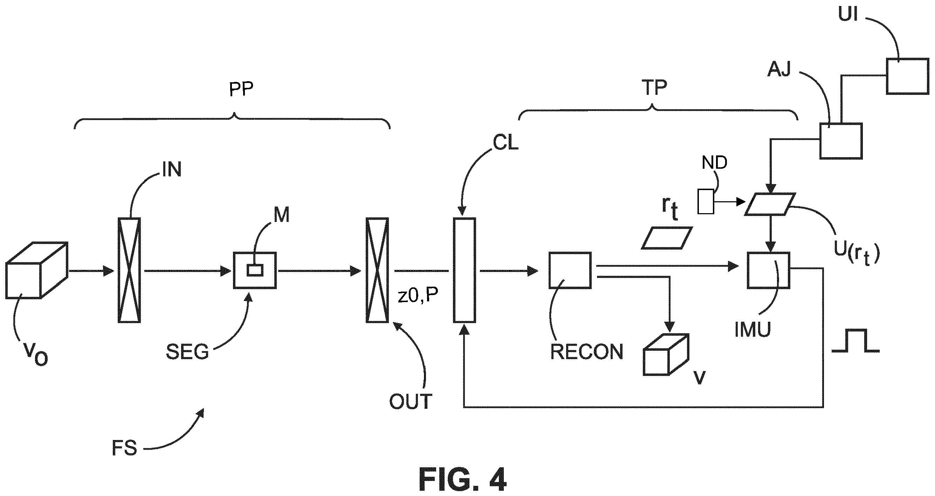

The facilitator system FS is preferably operable mainly across the two phases, the preparatory phase PP and the target phase TP, and is now described in more detail in the block diagram of Fig. 4 to which reference is now made.

The initial surview image V0 reconstructed from projection imagery acquired at lower image quality (lower dose) λ0 than the dose to be used for the diagnostic projection imagery λ for the later target volume V, is received at an input port IN.

A segmenter component SEG is used to segment the volume V0 for a location, such as an anatomical landmark, that is to serve as the monitoring region m-ROI for arrival of bolus at the target anatomical feature TAF. Preferably, the segmentation operation is machine learning based so includes a machine learning model M, previously trained on training data as will be explained below in more detail. Optionally, model may be further trained to also segment the surview volume V0 for target anatomical TAF, or the facilitator FS may include a different ML model, trained to segment for the target anatomical TAF, as mentioned above in connection with the auto-plan. However, as said, the location of the target anatomical TAF is general assumed to be known herein, and can be specified by whichever means, manually, or by ML or any other way.

The segmentation (result), that is the sub-volume s, may then be output at (internal) output port OUT and is then made available for the subsequent monitoring phase, but may also be used for other purposes. For example, it may be desirable to immediately, or later, visualize for cross-check purposes, the segmentation in surview volume. This, visualizer VIZ may be instructed by system to render to combined visualization of volume V0 (in a selectable view, axial, sagittal, etc), with the segmentation s suitably marked up there, by color or grey value encoding of its volume and boundary, as an overlay, etc, or by any other visualization technique, as desired. For visualizing the segmentation as such, any suitable view of the volume Vo may be rendered, sagittal, coronal, axial, as required. Alternatively, true 3D rendering is down, optionally with rotation (automatic or upon user interaction with pointer tool for example), so that user can quickly visually examine the segmentation from manifold directions and ascertain how it fits into the surrounding anatomy - which, as said, is at this time only shown, if at all, in weak contrast owing to the low dose expended on the surview V0. As observed before, the shape, orientation, etc, of the segmentation s is that of the surrounding anatomy, such as a section of a blood vessel.

The segmented sub-volume s, representative of the correct landmark associated with the m-ROI, may be processed at the output interface to produce the reference location Z0. For example, a centroid or other geometrical reference point P within or on the sub-volume s may be used to define the reference location for the tracker imagery RT. For example, the z coordinate of the geometrical reference point may be taken as the reference location z0 for the m-ROI.

The reference location z0 may optionally, but preferably, be used initially to synthesize from the surview volume V0 a "locator" (or "proto-type") tracker image r0, in a plane that passes through the reference location z0. This proto-type tracker image r0 may be optionally visualized by visualizer VIZ and displayed on display device in any desired view, such as axial, coronal, sagittal. Again, this provides a useful cross-check feedback for user to check whether the computed segmentation is in line with user's expectation. The reference point may be marked up visually in the displayed locator tracker imager r0 and/or it is the neighborhood that is so marked up.

It is of note that dose can be saved, as no separate projection data acquisition is required for the proto-type tracker image r0 as this can be synthesized from the surview volume V0 purely computationally. Specifically, the neighborhood U for use in the "live" tracker imagery as for live monitoring of bolus arrival can already be defined on this proto-tracker image synthesized off the already available surview 3D image V0. No dose needs to be incurred for determining the neighborhood U which represents the spatial extension of the monitoring region of interest m-ROI as representable in the tracker imagery. What is more, both the reference location and the neighborhood U determination can be done in a single step solely based on the surview image V0 and the so synthesized locator tracker image r0. This is more convenient and quicker for the clinical user, and this 1-step operation can still be easily integrated into existing CT workflows as practiced in medical facilities worldwide. It will be understood that use of the locator tracker image r0 is not dependent on its displaying and such displaying is indeed not required herein in all embodiments for the purpose of neighborhood U definition as described in the single step setup.

The reference location z0 so derived from the segmentation s may be used by a controller CL to instruct imager to commence acquiring the series of low dose tracker projection imagery $\lambda''$ around the reference location Z0 at a suitable (for example, user adjustable) sampling rate. For some or each set of such tracker projection imagery $\lambda''$, the reconstructor RECON may be instructed through interface CL to reconstruct the series of tracker imagery r(t) from the series of low dose tracker projection imagery, and each tracker image may then be displayed on display device DD in a video feed in sequence as they are computed. However, such displaying is optional, and the monitoring operation (on which more details below) for bolus arrival can be done without displaying. The subsequent tracker images r(t) so reconstructed are essential copies of the synthesized locator tracker image r0 in terms of location, but are updated versions of r0, with changes in image values due to gradual build-up of contrast material.

Again, the image quality of this series of tracker projection imagery $\lambda''$ acquired during the monitoring phase is of lower quality (with lower dose) than the projection imagery $\lambda$ to be acquired at the later diagnostic phase. For example, the image quality may be similar to the one of projection imagery $\lambda 0$ for the initial surview volume V0, or may be lower still, or somewhat higher, with radiation dosage adjusted accordingly and acquisitions triggered.

In each tracker image $r = r(t) = r_t$, a suitably sized image neighborhood $U = U_{t,s} = U_{t,P(s)}$ (an image subset) around the reference point $P = P(s)$ in the segmentation s is defined automatically by a neighborhood definer ND to define the m-ROU in each or some instance of tracker image. Thus, in some or preferably in each, of the tracker imageries $r(t)$, the neighborhood $U_t$ is defined around the same reference point P, related to the same reference location z0. Each tacker image $r_t$ may be an axial slice. The increased build-up of contrast material at the m-ROI (which is in the FOV of the imager IA) will be offset for the low dose setting when acquiring tracker projection imagery for the series tracker images $r_t$.

The reference point P may be expanded into such a neighborhood U of any desired shape and size. For example, the neighborhood $Ut$ may be defined as circle, ellipse or other shape, placed around this detected centroid P in each tacker image $r_t$. The size of the circle could be pre-defined or set automatically so that it is contained entirely and preferably conforms the anatomy to which the segmentation refers to, such as a target section of the target vessel. User may adjust any one of size, position, orientation of the system-proposed neighborhood $Ut$. Alternatively, and for simplicity, the neighborhood around P may be expanded to a default sized circle, ellipse etc, as pre-defined, such as 20mm diameter, etc. User may then request through UI to adjust size, either interactively, or by keying in data via a keyboard, or by selecting from a list of sizes (10mm, 5mm, 2mm, etc), etc.

An In-image monitoring unit IMU is configured to perform the above-mentioned bolus arrival based on examining evolution of image values in the respective neighborhoods $U_t$. To this end, in - image monitoring unit IMU may examine each voxel value within the confines of neighborhood $U_t$, or may examine for example only averages over such values, etc. In-image monitoring unit IMU may use any suitable thresholding policy to monitor the image values representative of the contrast agent concentration through the series of tracker imagery $r_t$ in the respective neighborhood $U_t$.

The policy may include a predefined trigger-condition formulated in terms of threshold magnitudes or gradients, or a combination thereof. Once the trigger-condition is found by in-image monitoring unit IMU to be met based on the examined evolution of in-image values across neighborhoods $U_t$, a control signal is issued to controller CL. Controller CL then operates to cause triggering the diagnostic projection imagery acquisition at diagnostic quality. Controller CL may then instruct reconstructor RECON to reconstruct the target image V from the diagnostic projection imagery λ now acquired.

The acquisition trigger signal issued by the in-image monitoring unit IMU may include again a request for adaptation of the imaging geometry to ensure that the to be acquired diagnostic projection imagery λ fully covers the area of the targeted anatomical feature TAF. However, as mentioned above, in most and preferred cases, the target anatomical feature will be sufficiently close to the monitoring region m-ROI, or the FOV is so chosen from the outset, so that no such adaptation is required (which is preferred herein), for example, if the section along the rotational axis Z covered by the scan ("scan box") is broad enough, which may depend on the width of the detector XD in Z-direction for example.

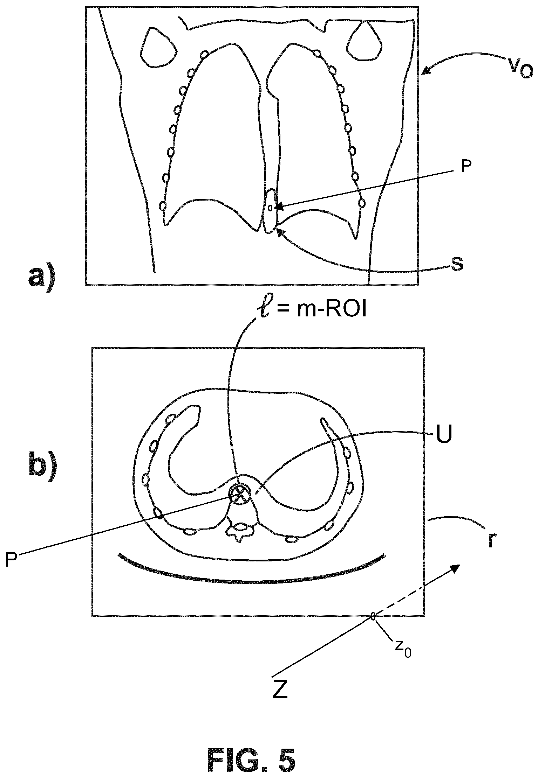

Fig. 5 is an illustration of the data generated by the facilitator system FS. Specifically, pane a) shows a coronal view of the 3D surview volume V0, with the segmentation s for the monitoring region highlighted therein, with the reference point P (such as centroid) taken from the sub-volume/region marked out by segmentation s. Thus, a corresponding section of the sub-volume segmentation s is illustrated in coronal view pane a). The segmentation may be have a different appearance/geometrical structure when rendered in another view of V0.

Based on the segmented sub-volume, the reference location $\ell = z0$ may be defined. For example, output interface OUT may be operable to determine (via a localizer component/functionality) the reference point P as centroid or other point of the area mapped out by segmentation s, and by taking the z-coordinate of said point P. Once the reference location $\ell = z0$, this determines the location in 3D of the monitoring region m-ROI and of the tracker image slices r, which are chosen to pass through the reference location $\ell = Z0$ or reference point P. Reference location also determines where the tracker projection data is to be acquired. Imaging geometry eg, one or more of gantry position, bed position) may need to be adapted to ensure projection data around z0 can be acquired. The triggering of the tracker projection data acquisition is in generally done by the user as function of commencement of when bolus administration device ADA is activated. To be on the safe side, one may trigger tracker projection data acquisition on start of bolus administration, or shortly thereafter, for example once the whole bolus volume has been so administered.

Monitoring by in-image monitoring unit IMU takes place for voxels in the neighborhood U, function of s and P, as illustrated in pane b). Indeed, the facilitator system FS may be operable to call the visualizer VIZ to visualize the segmented surview image Vo any desired view such as in sagittal, coronal, or axial, as along any other reformat direction. The reference location P around which the monitoring neighborhood U is expanded is marked by a cross ("X") in Fig. 5, and such may be indeed visualized in embodiment.

The neighborhood U may be defined as an ellipse, circle, or any other geometrically suitable structure. The spatial dimensionality of the neighborhood U is in general 2D as it is defined in the tracker imagery which are in general 2D slice images. However, if required, neighborhood $U$ may be a rendered as a 3D such as an ellipsoid, ball, etc, if the tracker images $r$ are themselves 3D, such as slabs rather than slices, and such 3D view is wanted.

As mentioned earlier, the computation of the segmentation and the monitoring neighborhood U around the reference location L may computed automatically, for example by using the machine learning model M or any other. IN embodiments, it is the segmentation that is ML computed, whilst the determination of P(s) and the neighborhood U it can be done by geometrical analytical methods. If the neighborhood U size, orientation is to be constrained anatomical boundaries between tissue types, or vessel boundary or any other such anatomically driven constraints, classical analytical segmentation approaches such as region-growing methods, model-based segmentation and other may be used, or indeed a separate ML segmentation (different from model M of finding anatomical landmark s) may be used in embodiments.

However, user interaction in a feedback loop on the results is also envisaged herein, where an adjuster AJ is operable to adjust any one or more of the reference location P, the segmentation s, or indeed the size or rotational shape of the monitoring region $U$ that maps out the monitoring region m-ROI. For example, a user interface UI, such as a graphical user interface GUI, may be used for example where visualizations as an exemplary shown Fig. 5a),b), may be rendered. The visualization may be configured with interactive functionality: for example, user may request such adjustments by touch-screen action, or by using a pointer tool (stylus, comp-mouse, etc) to adjust the size of the neighborhood for example, the position, shape or size of the segmentation, or reposition the reference location P by clicking, pinching or any other user interaction, gesture based, or other.

It will be understood that if the segmentation or the reference location P is adjusted by the user based on an initially suggested segmentation and reference location, the associated neighborhood U is then re-computed in response to such adjustments. For example, adjustment of the segmentation may cause a re-computation of the reference location as the centroid for example may change if the segmentation volume is changed which in turn will trigger a new neighborhood U being computed around the updated location. Thus, any change of any of the elements of z0, U, P, s, may trigger a re-determination of the remaining ones of such elements. In addition, or instead, modifications in the tracker imagery at any time may be done by the user, for example by adjusting the location reference location, the neighborhood U is caused to be co-adjusted. In some embodiments, adjustment of the neighborhood U may equally trigger re-computations of the segmentations and/or reference location, and so on. Thus, the user input is embedded and acted up in a dynamic and preferably next to real-time manner.

The example in Fig. 5 shows the organ of interest TAF being the kidney, whilst the monitoring region for the kidney may be chosen as the descending aorta diaphragm. Thus, depending on the target anatomy TAF, and hence imaging protocol, the position of the bolus m-ROI may change. For example, for target anatomy TAF/enhancements in the coronary arteries, the m-ROI is placed in the ascending aorta near the heart. For cardiac applications, mainly (but not exclusively) envisaged herein, suitable m-ROIs include any one or more of (a) aortic arch, (b) ascending aorta, (c) descending aorta mid-chest and (d) Pulmonary trunk. The proposed method may be applied to any (additional or alternative) anatomical landmark tracking locations m-ROI, as desired or prescribed in the exam protocol and/or the CT workflow.

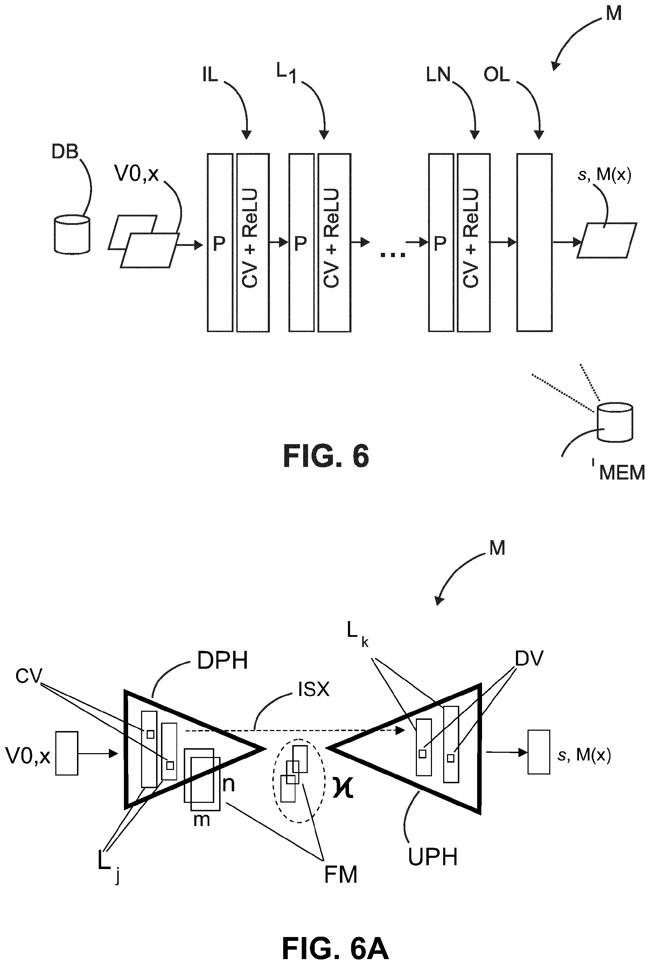

Reference is now made to Fig. 6 which shows an embodiment of the machine learning model M and as may be used by the segmentor SEG.

Specifically, Fig. 6 which shows components of a convolutional neural network CNN type model as is envisaged herein in embodiments, including convolutional filters CV to better account for the mentioned spatial correlation in the pixel data that makes up the input V, the surview image. The setup will be explained largely for post-training (deployment or testing), but the below applies also where the input is a training surview image x.

Specifically, Fig. 6 shows a convolutional neural network M in a feed-forward architecture. The network M comprises a plurality of computational nodes arranged in layers in a cascaded fashion, with data flow proceeding from left to right and thus from layer to layer. Recurrent networks are not excluded herein.

In deployment, or training, input data including surview volume V0 is applied to input layer IL. Thus, input data x=V0 is fed into model M at input layer IL, then propagates through a sequence of hidden layers L1-L3 (only three are shown, but there may be one or two, or more than three), to then emerge at an output layer OL as the training data output M(x), or the final segmentation M(V0)=s in deployment. The network M may be said to have a deep architecture because it has more than one hidden layers. In a feed-forward network, the "depth" is the number of hidden layers between input layer IL and output layer OL, whilst in recurrent networks the depth is the number of hidden layers, times the number of passes.

The layers of the network, and indeed the input and output map, and the input and output between hidden layers (referred to herein as feature maps), can be represented as two or higher dimensional matrices ("tensors") for computational and memory allocation efficiency.

The output layer OL determines the type of the model and this is preferably one of classification, as segmentation can be understood as a classification on voxel level for example. The output at output layer OL is thus configured to output a segmentation map, binary or continuous, which is a voxel-wise classification result on whether or not (or with what probability) each or some voxels represent part of an acceptable m-ROI for the given task. Thus, in terms of dimension and size (m x n x o) the classification map/segmentation map has the same size as the input surview volume V0, x. The output layer may be configured based on the soft-max function.

Preferably, the hidden layers include a sequence of convolutional layers, represented herein as layers L1 - LN-k, k>1. The number of convolutional layers is at least one, such as 2, 3, 4 or 5, or any other number. The number may run into double-digit Figs.

In embodiments, downstream of the sequence of convolutional layers there may be one or more fully connected layers, but this is not necessarily the case in all embodiments and indeed preferably no fully connected layer is used in the architecture envisaged herein in embodiments.

Each hidden Lm layer and the input layer IL implements one or more convolutional operators CV. Each layer Lm may implement the same number of convolution operators CV or the number may differ for some or all layers.

A convolutional operator CV implements a convolutional operation to be performed on its respective input. The convolutional operator may be conceptualized as a convolutional kernel. It may be implemented as a matrix including entries that form filter elements referred to herein as weights θ. It is in particular these weights that are adjusted in the learning phase. The first layer IL processes, by way of its one or more convolutional operators, the input data. Feature maps FM are the outputs of convolutional layers, one feature map for each convolutional operator in a layer. The feature map of an earlier layer is then input into the next layer to produce feature maps FMi, FMi+1 of a higher generation, and so forth until the last layer OL combines all feature maps into the output M(x), s. Entries of each feature map may be described as nodes. The last combiner layer OL may also be realized as convolution that provides the corrected front image.

A convolutional operator CV in a convolutional layer is distinguished from a fully connected layer in that an entry in the output feature map of a convolutional layer is not a combination of all nodes received as input of that layer. In other words, the convolutional kernel is only applied to sub-sets of the input volume V, or to the feature map as received from an earlier convolutional layer. The sub-sets are different for each entry in the output feature map. The operation of the convolution operator can thus be conceptualized as a "sliding" over the input, akin to a discrete filter kernel in a classical convolution operation known from classical signal processing. Hence the naming "convolutional layer". In a fully connected layer, an output node is general obtained by processing all nodes of the input layer.

The stride of the convolutional operators can be chosen as one or greater than one. The stride defines how the sub-sets are chosen. A stride greater than one reduces the dimension of the feature map relative to the dimension of the input in that layer. A stride of one is preferred herein. In order to maintain the dimensioning of the feature maps to correspond to the dimension of the input imagery, a zero padding layer P is applied. This allows convolving even feature map entries situated at the edge of the processed feature map.

In a preferred embodiment (see schematic inset Fig. 6A), a convolutional neural network CNN is used for model M that is configured for multi-scale processing. Such models include, for example, the U-unit architecture or its cognates, as described by O. Ronneberger et al in "U-Net: Convolutional Networks for Biomedical Image Segmentation", available online on arXiv repository under citation code arXiv: 1505.04597 (2015).

In this or similar architecture multi-scale capable NN models, de-convolutional operators DV are used. In more detail, such multi-scale enabled architecture of model M may comprise, in series a downscale path DPH of layers and, downstream thereof, an upscale path UPH of layers. Convolutional operator with varying stride length, gradually decrease feature map size/dimension (m x n) with passage through layers Lj in downscale path, down to a lowest dimensional representation by feature maps, the latent representation $\kappa$. The latent representation feature maps $\kappa$ have their dimensions than gradually increased with passage through the layers Lk of the upscale path UPH, back to the dimension of the original input data x. In this, the output is the segmentation s represented as map so has the same size (3D) as the input surview volume VO, x. This dimensional bottleneck structure has been found to cause better learning as the system is forced to distil its learning down to the simplest possible structure as represented by the latent representation.

Additional regularization channels may be defined where intermediate output (intermediate feature map) from a scale level in the down-scale path fed into a corresponding layer at a corresponding scale level in the up-scale path UPH. Such inter-scale cross connections ISX been found to foster robustness and efficiency of learning and performance.

The multi-scale processing of the model of Fig. 6A or similar is of particular benefit herein. Specifically, as the contrast in the initial 3D surview image is poor, yet the structure of interest such as the monitoring ROIs m-ROIS/landmarks (and optionally the target anatomical feature) may still be sufficiently segmented for because of the multi-scale processing where even minute contrast gradients may be sufficient for the multi-scale model M to find the desired segmentation s of the m-ROI. In addition, as will be explored in more detail below in relation to the training data generator system TDGS, diagnostic training imagery of sufficient contrast may be used to train the model M.

It will be understood that the monitoring location s for which the initial surview V0 is segmented for may be different for different imaging tasks. Thus, each imaging task or purpose defines the target anatomical feature TAF, which in turn, by clinical knowledge, defines the suitable (one or more) monitoring regions m-ROI as anatomical landmarks associated with the target feature TAF for that medical imaging task. Thus, different models Mj may be trained, at least one for each given imaging task/protocol/specification. Thus, each model Mj in the set of models so obtained {Mj }is configured to segment for different monitoring regions as associated with i) different target anatomical features TAF or ii) with different tasks for the same TAF, etc. More specifically, the set of models{Mj }may be stored and managed as a bank or library of different such trained models Mj. A database management system MEM' may provide a platform for automatic or user-initiated query to retrieve or otherwise access the correct model Mj' for a given task j'.

A specification of the image task j' to be performed may be supplied by the user prior to imaging. The specification may be a file, such as an XML file, a message, or any other. For example, user may select by a selective or menu structure, etc, in a GUI or other interface, the image task j', An event handler (not shown) that uses the selection signal to provide access to the trained model Mj' associated in with this task', and input at input port IN is the fed into the model Mj' so selected. For example, and in more detail, a specification (such as an xml file, a flag, etc) of the task j' is provided, alongside the surview image V0, and both are received at input port IN. The specification allows selection of the corresponding machine learning model Mj, or more generally of the associated segmentor SEGj, that is configured to find the suitable landmarks for the monitoring regions m-ROI, associated with the target anatomical feature TAF and/or task j' at hand.

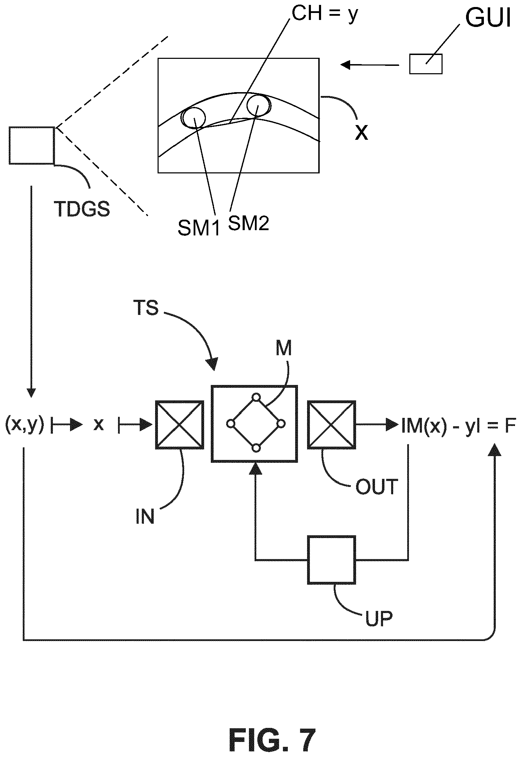

Reference is now made to Fig. 7 which shows a training system TS for training, based on the training data, the ML model M for use by segmentor SEG.

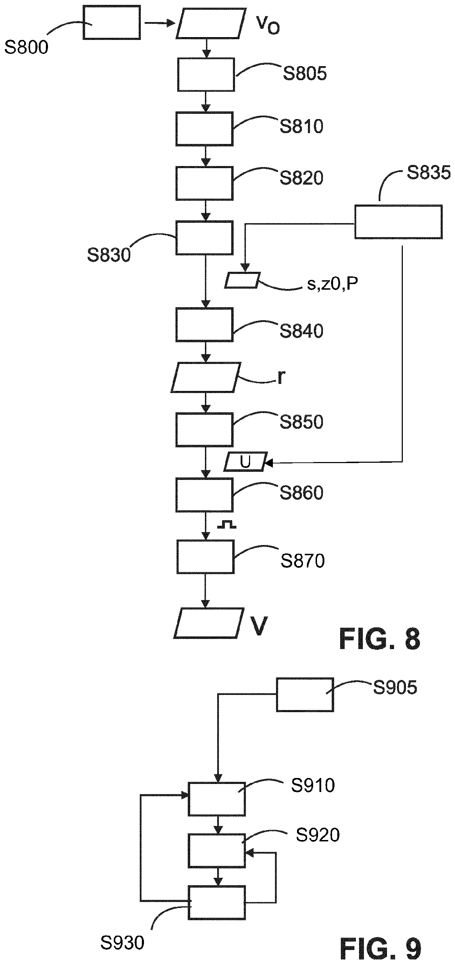

The training data preferably includes annotations down to voxel-level, to ensure the desired level of accuracy envisaged herein. A training data generator system TDGS may supply labeled training instances (x,y) where x stands for a historic surview image drawn from an image database, and y designates its ground truth label/annotation. The label may be a voxel map that singles out those voxels that together represent an acceptable definition for an m-ROI. Labelling can be done in the 3D volume of x, or in one of its views such as coronal, as desired. The training data generator system TDGS may be arranged as an interactive user interface system (UI), that supports user in the annotation task. For example, training data generator system TDGS may be configured to allow user to define seed markers SM1-2 in the training input surview volumes x. Such seed markers SM1-2 may be used to define a convex hull CH which automatically generates the map. The seed markers, eg, a pair (or more) circles or other geometrical Fig, are set by expert via a graphical user interface GUI of the training data generator system TDGS into the training input surview image x. This is explained in more detail below at Fig. 9.

As observed earlier, the surview input imagery x, as indeed any surview image, has very poor contrast, and it may not be always possible, even for a human expert, to annotate for the ground-truth m-ROIs reliably, or at all. In such situations, the training data generator system TDGS may be operable to generate the pairs of training data (x,y) as follows, based on noise simulation. A set of extant historic 3D diagnostic images (spectral or non-spectral imagery) of the general body part of interest, for which the model is to be trained, is localized in a database query in medical database, such as in a PACS, or other. In such diagnostic images, the annotation can be achieved easily by the clinical expert to define location and extent of the m-ROIs. Then, the high IQ (image quality) of the diagnostic images, that were used for the annotation, is artificially reduced by simulating noise and adding this noise to the diagnostic images, thereby simulating the low dose effect in surview imaging, and thus obtaining artificially generated samples that represent, in good approximation, instances of surview volumes. The noise corrupted 3D image samples thus may serve as the training input x, whilst the annotation on the high-quality imagery serves as the associated ground truth y. Thus, the training data generator system TDGS may include a noise simulator and a noise adder to generate as many pairs of training data as needed, along the lines described above.

Training system TS is configured to train ML model M based on such training data TD = {(x,y)}. The training data is optionally provided by the training data generator system TDGS.

In supervised settings, the training data may comprise pairs k of data (xk, yk). The training data comprises for each pair k, training input data xk and an associated target or ground truth yk. The training data is thus organized in pairs k in particular for supervised learning schemes as mainly envisaged herein. However, it should be noted that non-supervised learning schemes are not excluded herein. x, and y are as defined above.

In training phase, an architecture of a machine learning model M, such as the CNN network in Fig. 6 below is pre-populated with initial set of weights. The weights $\theta$ of the model NN represent a parameterization $M^\theta$, and it is the object of the training system TS to optimize and hence adapt the parameters $\theta$ based on the training data (xk, yk) pairs. In other words, the learning can be formulized mathematically as an optimization scheme where a cost function F is minimized although the dual formulation of maximizing a utility function may be used instead.

Assuming for now the paradigm of a cost function F, this measures the aggregated residue(s), that is, the error incurred between data estimated by the neural network model NN and the targets as per some, or all, of the training data pairs k:

$$argmin_\theta F = \sum_k |M^\theta(x_k), y_k| \qquad (1)$$

In eq (1) and below, function M() denotes the result of the model NN applied to input x. The cost function may be pixel/voxel-based. For a classification task, as envisaged herein in some embodiments, the cost function F sum in (1) may be based on cross-entropy, weighted cross-entropy or Negative Log Likelihood (NLL) divergence or similar. As a further alternative, the cost function may be based on the Dice coefficient function that measures differences between sets in terms of the size of their overlap (intersection), in which case the input training output and the label segmentation map are compared as sets. The Jaccard index function may also be used.

However, it may be possible to express the segmentation task as one of regression rather than as a classification, in which case the cost function may be based on a squared Euclidean distance between the predicted map M(x) and the label map y. Thus, L2 loss function may be used, or even an L1 loss function in some cases.

In training, the training input data xk of a training pair is propagated through the initialized network M. Specifically, the training input xk for a k-th pair is received at an input IL, passed through the model M, and is then output at output OL as output training data Mθ(x). A suitable distance measure | · | is used such as a p-norm, squared differences, or other, to measure the difference, also referred to herein as residue, between the actual training output Mθ(xk) produced by the model NN, and the desired target yk. However, the distance measure | · | is not necessarily based on an underlying norm definition. The notation "| · |" as used herein is a generic notation referring to any suitable distance measure of any kind.

The output training data M(xk) is an estimate for target yk associated with the applied input training image data xk. In general, there is an error between this output M(xk) and the associated target yk of the presently considered k-th pair. An optimization scheme such as back-propagation or other gradient based methods may then be used to adapt the parameters $\theta$ of the model NN so as to decrease the residue for the considered pair (xk, yk) or a subset of training pairs from the full training data set.

After one or more iterations in a first, inner, loop in which the parameters $\theta$ of the model are updated by updater UP for the current batch of pairs { (xk,yk) }, the training system TS enters a second, an outer, loop where a next batch of training data pair {xk+1, yk+1} is processed accordingly. Whilst it is possible to loop in the outer loop over individual pairs until the training data set is exhausted, it is preferred herein to loop over sets of pairs instead (namely over the said batches of training pairs) at once). Iteration in the inner loop is over parameters for all pairs making up the batch. Such batch-wise leaning has been shown to be more efficient then proceeding per pair. In the following, the subscript notation "k" to indicate individual instances of training data pair will be largely dropped to disencumber notation.

The structure of updater UP depends on the optimization scheme used. For example, the inner loop as administered by updater UP may be implemented by one or more forward and backward passes in a backpropagation algorithm. While adapting the parameters, the aggregated, for example summed, residues of all the training pairs in a given batch are considered up to the current pair, to improve the objective function. The aggregated residue can be formed by configuring the objective function F as a sum of squared residues such as in eq. (1) of some or all considered residues for each pair. Other algebraic combinations instead of sums of squares are also envisaged.

GPUs may be used to implement the training system TS for enhanced efficiency.

Whilst voxel-wise training is preferred herein, this is not necessarily so in all embodiments, as a more coarse-grained training on patches or tiles (subsets of voxels) is not excluded herein on order to safe CPU time and memory, or to make training faster.

Reference is now made to flow chart in Fig. 8, which shows steps of a computer-implemented method to carry into practice the above-described facilitator system FS. Flow chart Fig. 9 relates to aspects of training the model, and as such is related to training system TS. However, the flow chart(s) below may also be understood as a teaching in its own right, not necessarily in all embodiments tied to the above. However, in some embodiments the below is indeed to be understood in the context of the above-described embodiments,

Turning first to Fig. 8, the method is computer-implemented, and allows facilitating contrast agent assisted imaging protocols, such as angiography, in particular for imaging sessions of the tomographic type, such as angiography CT or C-arm. That is, X-ray based modalities, such as radiography, CT, C-arm, mammography, tomosynthesis, etc, are all envisaged herein in different embodiments. Attenuation imaging is mainly envisaged, but that is not to say that other imaging techniques, such as phase-contrast, dark-field, etc, are excluded herein. They are not. Also, non-X-ray based imaging modalities that may use contrast agent in some form, such as MRI, nuclear imaging, etc, are also envisaged herein, and the proposed method and system may also be used with benefit there.

At step S800 the 3D survie image V0 at a lower image quality than of the subsequent diagnostic target volume V is obtained. This may be done by acquiring projection imagery $\lambda'$ in a first set at lower dose than the dose for the diagnostic projection imagery $\lambda$ to be used later for the diagnostic volume V. The low dose projection imagery $\lambda'$ in projection domain so acquired is then reconstructed in image domain by a tomographic reconstruction algorithm, such as FBP, iterative, algebraic, or any other.

At step 805 the survie image V0 is received by the computing system(s) on which the method is to be practiced.

At step S810 the low dose (low IQ) 3D survie volume V0 is segmented for one (or more) monitoring region m-ROIs, such as an anatomical landmark that is associated with a target anatomical feature TAF. The target anatomical feature TAF is or defines the medical purpose of the imaging. The purpose or the target anatomical feature TAF may be specified in a specification, that may also be received at step S805, alongside the 3D survie volume V0 in embodiments. However, the specification may be preset or is otherwise pre-determined by context, so the step S805 of receiving this specification need not be an explicit operation, but may be implicitly provided in the computing environment in which the method is practiced, or in any other way or manner.

The specification, in whichever form provided or received, explicitly or implicitly, may determine a specific type of segmentor or segmentation algorithm to be used for step S810. The segmentor or segmentation algorithm may be specially geared to find the specific associated landmark as monitoring region in low dose survie imagery, given the purpose or nature of the target anatomical feature TAF. Segmentation at step S810 may be ML-based, such as based on a trained NN model, such as a CNN, preferably configured for multi-scale processing. Any other ML model capable of such segmentation is envisaged herein, and so are more classically configured segmentation techniques, not ordinarily considered ML, such as region-growing, or preferably (shape-)model-based segmentations ("MBS"), etc. However, ML based method have been found to cope well with the generally low IQ in survie imagery.

The FOV for the survie image VO at S800 may be a priori, or based on ML, so chosen that the intended m-ROI(s) and the target anatomical feature TAFS are roughly known to be covered by the imaging based on general medical knowledge, although prior to step S810, the exact location of the m-ROI is not known.

At step S820, the segmented landmark for the monitoring region m-ROI is made available. The segmentation s for the monitoring region m-ROI is a sub-volume (a sub-set) of the survie volume V0. Thus, the segmentation is also of 3D character. Preferably, the segmentation s of monitoring region m-ROI conforms in shape and extent to the surrounding anatomical tissue boundary that it interfaces.

In general, the bolus monitoring region m-ROI is based on the segmentation s. The monitoring region m-ROI has a location (reference location z0), and a spatial extension in relation to the tracker imagery. The tracker imagery is to be obtained to monitor for bolus arrival. Each, the location and the extent of the m-ROI is to be determined as will now be explained in the following steps.

Tuning first to the reference location z0 for the m-ROI and the tracker imagery rt, this is determined at step S830, based on the segmented region s. For example, a reference point P of the segmentation s, such as centroid, may be computed of the 3D sub-volume, as defined in the segmentation s. P's z-coordinate in image domain may be used as the z-position for the tracker imagery r(t) to be obtained, thus defining the reference location z0.

In general, the reference location is located along the imager's rotation axis Z, and this is where the tracker projection imagery is to be acquired, and this where in image domain the tracker imagery r(t) is located.

As mentioned, the reference location z0 of the segmentation defines the location (as opposed to its spatial extent) in image domain of the monitoring region of interest m-ROI. The use of segmentation s's centroid P as described above, being merely one example. Other ways of defining the location of the m-ROI may also be envisaged herein, such as drawing the envelope of the segmentation structure s, etc. Alternatively, P is defined as the center-point of the largest circle/ellipse that fits within the area marked out by the segmentation. However, pinning the m-ROI down to a single coordinate (such by using centroid P) may be advantageous in terms of computational responsiveness and memory resources. Based on the segmentation s, any other manner of computing reference location z0 may be used instead, and this may involve more than one such reference point P.

At step S840, the tracker imagery *rt* is obtained. This may include acquiring a series overtime sets of tracker projection imagery $\lambda$" at a given sampling rate around the reference location, again at a lower dose than that required for the diagnostic image later. The tracker imagery rt is then reconstructed from the said sets, as a respective axial slice per time instant t, each in the plane passing through the reference location z0 as computed in step S830. Formats other than axial may be used instead, defined along any axis as required and as obtainable by a reformatting algorithm.

Preferably, administration of contrast agent may determine when in step S840 the acquisition of tracker projection imagery is to commence. This can start before or shortly after CA administration. Care should be taken not to "miss" the point where CA starts accumulating at the m-ROI.

At step S850 the extent of the monitoring region m-ROI as per some or each tacker image *rt* is defined.

Preferably, this is done at step S850 by defining a respective neighborhood U around the (one or more) reference point P in some or each instance of tracker imagery: $P \in Ut \subset rt$.

Preferably, instead of defining the neighborhood U in tracker imagery rt, this may be defined instead already beforehand in a locator image that may be synthesized off the surview volume V0, before "live" tracker imagery is obtained. That is, no new acquisition is required to define neighborhood U. The neighborhood so defined on the synthesized locator tracker image r0 may then be used over and over in the live tracker imagery instances reconstructed from newly acquired tracker projection data at step S840. Thus, step S850 can be done before step S840 of obtaining the live tracker imagery rt for monitoring.

The neighborhood U may, for example, be a respective ellipse, square, or of any other geometrical form or shape. Thus, each instance of the tracker imagery has the neighborhood Ut defined therein around the same reference location P.

Preferably the neighborhood U is so defined to respect anatomical tissue boundaries. For example, U may be so defined such that it does not exceed beyond image representation of blood vessel's walls in which the bolus is to travel and as captured in the respective instance of the tracker imagery rt. The neighborhood may be expanded as large as possible, so long as it remains within the image representation of the said vessel's wall. The neighborhood U needs to be determined only once, for example on the locator tracker image or the first one-line tracker mage, and this can then be reused for all follow up tracker images. The same applies to the reference location z0, as this needs to be determined once based on the segmentation s. If views are changed (eg from axial to sagittal or other), any of the above components s, *U, P*, etc, may need to be co-transformed accordingly.

Preferably, reference location definition z0 at step S830 and neighborhood *U* definition-step S850 based on locator image r0 are done in one procedural step, with steps S830, S850 merged, as both rely merely on surview image *V0* information, including the segmentation s therein.

At step S860 the series of tracker imagery is monitored for the arrival of the contrast agent. This may be done by thresholding or any other policy of analyzing image values in the neighborhood U around image location z,P in the tracking imagery.

Once the contrast agent concentration is deemed sufficient based on the monitored image values in the neighborhood U at step S860, a control signal may be issued to obtain the target volume TAF at step S870 at full dose, the location of which is, as mentioned, assumed known and included in the current FOV as used for the tracker imagery rt. Thus, as the FOV was chosen appropriately wide for the tracker projection data acquisition, the same FOV can be used for acquiring the diagnostic image V. Thus, step S870 may include, upon receipt of the trigger signal as per step S860, acquiring the diagnostic projection imagery $\lambda$ at a higher diagnostic dose at the location of the anatomical feature of interest TAF, from which projection imagery $\lambda$ the target volume can then be reconstructed.

At an optional, but preferred, step S835, a user interaction request may be received that requests modification of any one or more of i) the segmentation s, ii) the reference location z0 derived therefrom, iii) the reference point P, iv) and/or the monitoring neighborhood U in the tracker imagery as a function of the segmentation and/or location z0.

A modification of one or more of the components i)-iv) may trigger a corresponding adaptation of the one, more than one, or all of the other remaining components i)-iv).

Further optionally, the above-described method may include various displaying operations.

For example, optionally, prior to step S840, the locator tracker image that is synthesized off the surview volume may be displayed to user as a quality check, before the tracker imagery based on the newly acquired tracker projection imagery is displayed and indeed acquired.

The neighbored U of the m-FROI in each instance of the tracker imagery may be outlined graphically, such as by color coding or by line style modulation (dashed, bold, etc) of the outlining curve.

The surview image V0 in any desired view may be displayed concurrently with the video stream of tracker imagery instances or with each such instance as a still image. In the surview image, the segmentation s may be visualized outlined in color or by graphically modulated outlining curve (nold, dashed, etc). In embodiments, a graphics display such as in Fig. 5a),b) is envisaged for display, in any arrangement of panes a),b) as deemed fit. Displaying on a single display device DD, or across plural display devices, is envisaged. Each of the described display option may be realized on its own in isolation, or in combination with any one or more of the other options, as requested by user.

Reference is now made to Fig. 9 which shows a flow chart of a method of training a machine learning model based on training data.

The method may include initial step S905 of generating such training data.

Generating machine learning training data may include voxel-level based annotation of existing surview imagery as may be obtained from medical databases of prior exams. A medical user/expert may be used to perform the annotations by using a suitable pointer tool, for example, a graphical user interface-based tool is preferable as this allows making the annotation operation more efficient.

Specifically, ground truth data y, or labels, for the individual bolus tracking m-ROIs can be annotated in the form of binary voxel masks in existing, historical 3D surview images x. The masks indicate the acceptable range along a vessel in which the bolus tracking m-ROIs can be placed. Instead of a binary map, use of continuous probability maps whose values vary in the unit interval are also contemplated herein.

One efficient way to annotate such a range is to place spheres at the beginning and end point of such an acceptable range, and to adapt the radius of the spheres to the size of the vessel as represented in the surview training image x. As mentioned above, imagery of diagnostic IQ may be used for annotation to define the y's, whilst artificially noised versions thereof are used as the associated x's. The range can then be calculated as the convex hull CH (see Fig. 7) connecting and surrounding the spheres SM1-SM3. Again, an interactive GUI setup that affords graphical drawing input may be preferred to implement this step, as mentioned above at Fig. 7, in connection with the training data generator system TDGS. The so defined acceptably region will then automatically indicate that all voxels that are covered by the hull are represent m-ROI voxels, unlike voxels outside the hull. The mentioned spheres SM1-SM3 so placed from thus seed markers for the convex hull CH. Other seed markers of other shapes may be used, depending in the image structures of interest.

At step S910 the training data is received in the form of a set (batch) of pairs (xk,yk), optionally as generated at step S905 or as retrieved from elsewhere. Each pair includes the training input xk and an associated target yk. xk is a 3D image surview volume and, yk is the annotated segmentation map.

At step S920, the training input xk is applied to an initialized machine learning model M to produce a training output.

A deviation, or residue, of the training output M(xk) from the associated target yk is quantified by cost function F (such as eq (1) above). One or more parameters of the model are adapted at step S930 in one or more iterations in an inner loop to improve the cost function. For instance, the model parameters are adapted to decrease residues as measured by the cost function. The parameters include in particular weights W of the convolutional operators CV and optional bias terms.

The training method then returns in an outer loop to step S910 where the next batch of pairs of training data is fed in.

In the said step S930, the parameters of the model are adapted so that the aggregated residues of all pairs in a given batch considered are decreased, in particular minimized. The cost function quantifies the aggregated residues. Forward- and in particular backpropagation or similar gradient-based techniques may be used in the inner loop. Back-propagation allows considering the prediction error to be used to adapt the parameters. Thus, for each batch of pairs considered, more information and better generalization capability can be extracted thanks to backpropagation.

More generally, the parameters of the model M are adjusted to improve cost function F. In embodiments, the cost function is configured measure the aggregated residues, such as int terms of (weighted) cross-entropy or Dice function or other cost function configured for classification/segmentation task purposes. In embodiments the aggregation of residues is implemented by summation over all or some residues for all pairs considered. The method may be implemented on one or more general-purpose processing units TS, preferably having processors capable for parallel processing to speed up the training.

The components of system SYS may be implemented as one or more software modules, run on one or more general-purpose processing units PU such as a workstation associated with the imager IA, or on a server computer associated with a group of imagers.

Alternatively, some or all components of system SYS may be arranged in hardware such as a suitably programmed microcontroller or microprocessor, such an FPGA (field-programmable-gate-array) or as a hardwired IC chip, an application specific integrated circuitry (ASIC), integrated into the imaging system IA. In a further embodiment still, the system SYS may be implemented in both, partly in software and partly in hardware.

The different components of system SYS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope. Such reference signs may be comprised of numbers, of letters or any of alphanumeric combination.

**Claims**

1.  System (FS) for facilitating contrast-agent based tomographic imaging, the system comprising, when in use:

    an input interface (IN) for receiving a specification for an imaging operation of an imaging apparatus (IA) of the tomographic type, and a 3D surview image volume (V0) obtained by the imaging apparatus (IA) of at least a part of a patient in a preparatory phase prior to a contrast agent assisted imaging phase;
    a segmentor (SEG) configured to segment for a monitoring region, m-ROI, in the 3D surview image volume, the said m-ROI being associated to a target anatomical feature as specified in the specification; and
    an output interface (OUT) to provide a reference location (z0) of the segmented m-ROI in the 3D surview volume for facilitating monitoring for presence of contrast agent in relation to the target anatomical feature.

2.  System of claim 1, comprising a controller (CL) configured to instruct imaging apparatus (IA) to acquire a first set of projection data ($\lambda''$) whilst contrast agent is propagatable in patient, and comprising a reconstructor (RECON) to reconstruct, based on the projection data and at a first image quality, sectional tracker imagery (r) in a plane passing through the reference location.

3.  System of claim 1 or 2, an in-image monitoring unit (IMU) configured to perform such monitoring based on the tracker imagery in an image-neighborhood ($U$) in the tracker imagery, the said image-neighborhood (U) based on the reference location.

4.  System of claim 3, wherein the said image-neighborhood (U) is defined in a synthesized tracker image (r0) synthesized from the surview image (V0), and such image-neighborhood (U) is then used by the in-image monitoring unit (IMU) for monitoring in the said reconstructed tracker imagery.

5.  System of claim any one of claims 2-4, the controller (CL) configured to instruct imaging apparatus (IA) to acquire, at a second image quality higher than the first image quality, a second set of projection data ($\lambda$) upon the in-image monitoring unit (IMU) issuing a trigger signal based on the monitored one or more image values in the image-neighborhood.

6.  System of any one of preceding claims, wherein the said reference location is based on at least one reference point (P) in relation to the segmentation (s) of the m-ROI.

7.  System of any one of preceding claims, including an adjuster (AJ) for adjusting any one of location, orientation and/or spatial extent of the said in-image neighborhood.

8.  System of claim 7, wherein the said adjuster (AJ) is operable in response to an instruction receivable via a user interface (UI) configured to allow a user to request adjusting any one or more of: i) the said in-image neighborhood (IU), ii) the reference location (z0), iii) the reference point (P), iv) the segmentation of the m-ROI.

9.  System of any one of the preceding claims, wherein the segmentor (SEG) is based on a trained machine-learning model (M).

10. Method for facilitating contrast-agent based tomographic imaging, comprising:

    receiving (S805) a specification for an imaging operation of an imaging apparatus (IA) of the tomographic type, and a 3D surview image volume (V0) obtained by the imaging apparatus (IA) of at least a part of a patient in a preparatory phase prior to a contrast agent assisted imaging phase;
    segmenting (S810) for a monitoring region, m-ROI, in the 3D surview image volume, the said m-ROI associated to a target anatomical feature as specified in the specification; and
    providing (S820) a reference location (z0) of the segmented m-ROI in the 3D surview volume for facilitating monitoring for presence of contrast agent in relation to the target anatomical feature.

11. Method of training, based on training data, the said machine learning model of claim 9.

12. Method of generating training data based on which training system (TS) of claim 11 is to train the machine learning model.

13. Imaging arrangement (IAR) comprising the system (SYS) of any one of the preceding claims, and one of more of: the imaging apparatus (IA) and a contrast agent administration apparatus (ADA) for administering contrast agent.

14. A computer program element, which, when being executed by at least one processing unit, is adapted to cause the processing unit to perform the method as per any one of claims 10-12.

15. At least one computer readable medium having stored thereon the program element of claim 14, or having stored thereon the machine learning model as per claim 9.

**FIG. 1**

FIG. 2

a)   b)   c)

**FIG. 3**

**FIG. 4**

a)

$\ell$ = m-ROI

b)

FIG. 5

**FIG. 6**

**FIG. 6A**

FIG. 7

FIG. 8

FIG. 9

Europäisches
Patentamt

European
Patent Office

Office européen
des brevets

# EUROPEAN SEARCH REPORT

Application Number

EP 23 15 1500

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/111457 A1 (SIEMENS MEDICAL SOLUTIONS [US]; SIEMENS AG [DE] ET AL.) 30 September 2010 (2010-09-30) * page 4, line 28 - page 11, line 13 * ----- | 1-15 | INV. A61B6/00 |
| X | WO 2020/094596 A1 (KONINKLIJKE PHILIPS NV [NL]) 14 May 2020 (2020-05-14) * page 7, line 19 - page 42, line 31 * ----- | 1-15 | |
| X | US 2019/231288 A1 (PROFIO MARK VINCENT [US] ET AL) 1 August 2019 (2019-08-01) * paragraph [0012] - paragraph [0077] * ----- | 1-15 | |
| A | US 2016/325040 A1 (FLOHR THOMAS [DE] ET AL) 10 November 2016 (2016-11-10) * paragraph [0030] - paragraph [0100] * ----- | 1-15 | |

TECHNICAL FIELDS
SEARCHED        (IPC)

A61B

The present search report has been drawn up for all claims

| Place of search | Date of completion of the search | Examiner |
|---|---|---|
| Munich | 13 June 2023 | Hooper, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 1500

This annex lists the patent family members relating to the patent documents cited in the above-mentioned European search report.
The members are as contained in the European Patent Office EDP file on
The European Patent Office is in no way liable for these particulars which are merely given for the purpose of information.

13-06-2023

| Patent document cited in search report | | | Publication date | Patent family member(s) | | | Publication date |
|---|---|---|---|---|---|---|---|
| WO 2010111457 | A1 | | 30-09-2010 | CN | 102361592 | A | 22-02-2012 |
| | | | | EP | 2410914 | A1 | 01-02-2012 |
| | | | | US | 2010249582 | A1 | 30-09-2010 |
| | | | | WO | 2010111457 | A1 | 30-09-2010 |
| WO 2020094596 | A1 | | 14-05-2020 | CN | 112969412 | A | 15-06-2021 |
| | | | | EP | 3649955 | A1 | 13-05-2020 |
| | | | | EP | 3876842 | A1 | 15-09-2021 |
| | | | | JP | 2022509016 | A | 20-01-2022 |
| | | | | US | 2021386389 | A1 | 16-12-2021 |
| | | | | WO | 2020094596 | A1 | 14-05-2020 |
| US 2019231288 | A1 | | 01-08-2019 | US | 2019231288 | A1 | 01-08-2019 |
| | | | | US | 2022117570 | A1 | 21-04-2022 |
| US 2016325040 | A1 | | 10-11-2016 | CN | 106108928 | A | 16-11-2016 |
| | | | | DE | 102015208202 | B3 | 22-09-2016 |
| | | | | US | 2016325040 | A1 | 10-11-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. M. MITCHELL.** Machine Learning. McGraw-Hill, 1997, 2

- **O. RONNEBERGER et al.** U-Net: Convolutional Networks for Biomedical Image Segmentation. *arXiv: 1505.04597,* 2015